Europäisches Patentamt

**European Patent Office**

**Office européen des brevets**

(11) Publication number: **0 415 650 A2**

# EUROPEAN PATENT APPLICATION

(21) Application number: 90309246.8

(22) Date of filing: 23.08.90

(51) Int. Cl.5: **C05F 3/06**

The title of the invention has been amended (Guidelines for Examination in the EPO, A-III, 7.3).

(30) Priority: 29.08.89 GB 8919511

(43) Date of publication of application:
06.03.91 Bulletin 91/10

(84) Designated Contracting States:
AT BE CH DE DK ES FR GB GR IT LI LU NL SE

(71) Applicant: GREEN LAND SYSTEMS LIMITED

2 Kiltinny Road, Coleraine
County Londonderry, Northern Ireland BT52
1SQ(GB)

(72) Inventor: Gornall, Leslie Keth
2 Kiltinny Road, Coleraine
Londonderry, BT52 1SQ, Northern
Ireland(GB)

(74) Representative: Sorrell, Terence Gordon et al
Fitzpatricks 4 West Regent Street
Glasgow G2 1RS Scotland(GB)

(54) **Gas impervious seal for process apparatus.**

(57) A process apparatus which may be used for biologically degrading waste material and which comprises a container having a peripheral channel 15 formed in an upper rim thereof. At least one closure member 18 has a downwardly projecting peripheral skirt 19 locating in said channel 15. The channel 15 has a liquid located therein to provide a gas impervious seal around the periphery of the closure member 18, in use.

Fig. 3

## IMPROVEMENTS IN AND RELATING TO PROCESS APPARATUS

The invention relates to process apparatus and more particularly, although not exclusively, to such apparatus, and methods of use thereof, for biologically degrading waste material, e.g. cattle effluent slurry.

In such apparatus and processes presently being used it is normal to utilise the container, within which the process is being performed, also as a storage container for the gases produced. This entails the provision of costly and unwieldy expansion chambers at the upper volume of the container and makes it extremely difficult, if not impossible, to provide any lateral extension of the size of the container.

It is an object of the invention to obviate or mitigate these disadvantages.

According to one aspect of the invention there is provided a process apparatus which comprises a container having a peripheral channel formed in an upper rim thereof and at least one closure member having a downward projecting peripheral skirt locating in said channel, the channel having a liquid located therein to provide a gas impervious seal around the closure member periphery, in use.

The container may comprise a tank and may be fabricated of concrete formed, in situ. The container may have one or more transverse channel section members located across the container with the channel(s) thereof communicating with said peripheral channel, a separate closure member being provided for each area defined by one or more channel section members and the communications peripheral channel with the skirts thereof depending downward therein.

The or each closure member may be formed with at least one opening therein for locating apparatus to be in communication with the interior of the container, and may be fabricated from corrosion resistant material.

According to a further aspect of the invention there is provided a closure/support device which comprises a part-spherical member for locating within a complementary part-spherical aperture in a support member and an equipment support platform located on a planner surface at a truncated upper portion of the part-spherical member.

A closure/support device of the next preceding paragraph may be located in one or each opening in the closure member of the apparatus of the next but one preceding paragraph, the closure member comprising said support member.

According to a further aspect of the invention there is provided a method of biologically degrading waste material which comprises locating such waste material within a container of an apparatus as defined in any of the next preceding five paragraphs and maintaining the pressure of gases produced by the method at a relatively low pressure by feeding such gases from an outlet of the container to storage vessel exterior thereto.

The foregoing and further features of the invention may be more readily understood from the following description of a preferred embodiment thereof, by way of example, with reference to the accompanying drawings, in which:-

Fig 1 is a schematic side sectional view of a process installation;

Fig 2 is a schematic plan view of the installation of Fig 1;

Fig 3 is a perspective view of a part of the installation of Figs 1 and 2 on enlarge scale, and

Fig 4 is a side sectional view of a closure/support device for use with the installation of Figs 1 to 3.

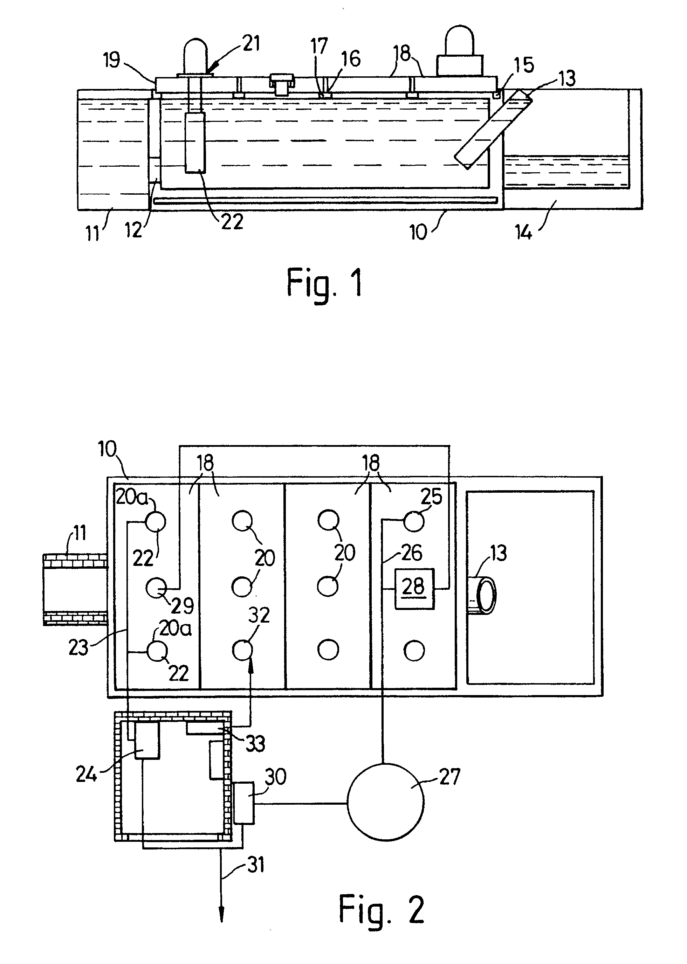

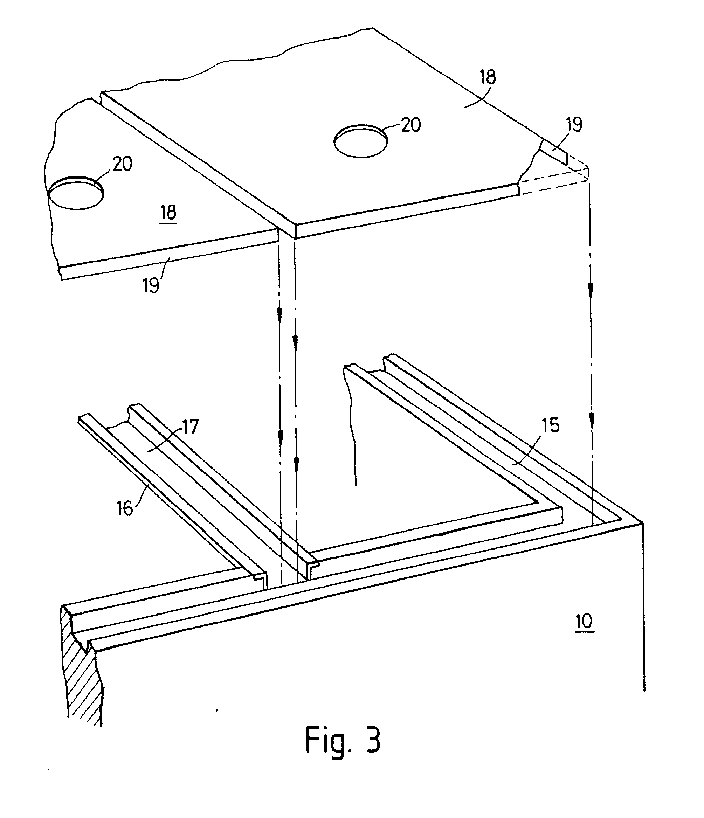

Referring now firstly to Figures 1 to 3 there is shown an installation for the biological treatment of cattle effluent slurry which comprises a treatment tank 10 having an inlet tank 11 connected thereto by a pipe 12 and an outlet weir 13 extending from the tank 10 to an output tank 14. The treatment tank 10 is formed with a peripheral channel 15 extending around the upper rim thereof and the top edge of the outer side wall may be lower than the top edge of the inner side wall to allow overflow outwardly due to overfilling of the channel 15 ie due to rainfall, in use. Transverse members 16 are provided with channels 17 formed therein which communicate with the peripheral channel 15 at each end. Four closure members 18 are located transverse across the treatment tank 10 and each have peripherally downward extending skirt portions 19 which locate within the channels 15 and 17.

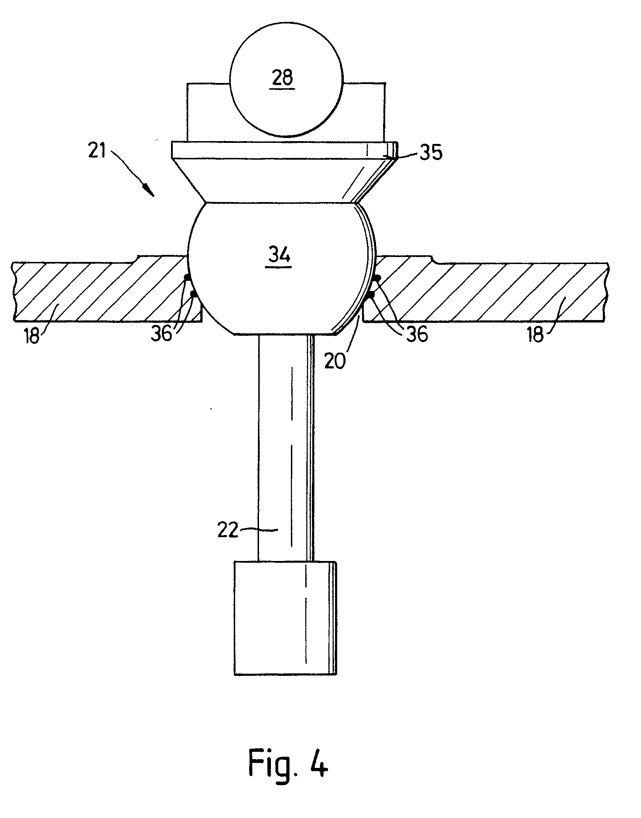

Each of the closure members 18 is formed with three openings 20 spaced thereabouts. Each opening 20 is closed by a closure/support device 21 which will hereinafter be more fully described with reference to Figure 4. Heaters 22 are arranged to depend from two of the closure/support devices 20A and are connected via a piping 23 to a boiler 24 (Fig 2). A gas outlet is connected via a piping 26 to a gas container 27 and via a compressor 28 to a rotary valve 29. An outlet from the gas container 27 is connected via a blower 30 to the boiler 24 and to an external take-off point 31. A water trap 32 is connected to be fed from a header tank 33.

In operation of the installation the channels 15 and 17 have water located therein which may be supplied and is kept topped up to a predetermined level via the water trap 32 from the header tank 33.

Slurry is fed into the input tank 11 and passes via pipe 12 into the treatment tank 10, the upper level of the slurry in the treatment tank 10 being maintained at a predetermined height by the weir 13, overflow from which enters output tank 14. The slurry is maintained at a predetermined temperature by the heaters 22 and gas collects above the upper level of the slurry and beneath the closure members 18. The gas is allowed to pass via gas outlet 25 and pipe 26 to the container 27 from which it can be removed via blower 30 to feed the boiler 24. The provision of the gas container 27 enables the gas pressure within the treatment tank 10 below the closure members 18 to be maintained at a low pressure for example 1/8th of an inch (3.2 mm) water gauge pressure, but each pressure being determined by the depth of the channels 15 and 17 and skirts 19 of closure members 18.

With such a treatment installation extremely, large masses of coagulated solid material can form within the treatment tank 10 and movement of such masses, caused for example by an agitator (not shown) can cause extreme damage to parts of the equipment depending into the treatment tank, i.e. the heaters 22.

Referring now to Figure 4 there is shown the detail of a suitable closure/support device 21 for location at each of the openings 20 in the closure members 18. The device 21 comprises a truncated spherical member 34 having a support platform 35 attached to or internally formed with the upper surface of member 34. The openings 20 are formed to be part-spherical and seals 36 or a liquid sealont are located between the outer surface of member 34 and part-spherical surface of opening 20. The compressor 28 is shown located on the support platform 35 and a heater 22 is shown arrangement should any large mass come into contact with the heater 22 the provision of the device 21 allows for rotating, upward or sideways movement of the heater 22 without damage thereto.

Hence by providing an installations having separate gas storage exterior of the treatment tank and by providing separate closure members with the simple gas impermeable liquid seals a relatively inexpensive installation kit can be provided which is easily transportable and installed which does not have the requirement of high pressure gas containment. Furthermore the treatment tank can be extended laterally without great difficulty. Although the treatment tank of the embodiment is shown to be rectangular such an arrangement is not essential and could for example be hexagonal to provide an easily honeycomb form of treatment tanks. The tanks may be formed of concrete, in situ, possibly with the channels lined with or formed of synthetic plastics material. Alternatively the complete tanks may be formed of synthetic plastics material. The closure members may be fabricated of a corrosion resistant material such as glass reinforced synthetic plastics, aluminium or canvasing resistant coated steel. The arrangement shown and described is suitable for aerobic or anaerobic digestion of waste slurries. However, with suitable modification to the input of the tank such apparatus may also be utilised for aerobic or anaerobic composting of solid or semi-solid waste materials.

## Claims

1. A process apparatus characterised by comprising a container (10) having a peripheral channel (15) formed in an upper rim thereof and at least one closure member (18) having a downwardly projecting peripheral skirt (19) locating in said channel (15), the channel (15) having a liquid located therein to provide a gas impervious seal around the closure member (18) periphery, in use.

2. An apparatus as claimed in claim 1 characterised in that the container (10) has one or more transverse channel section members (16) located across the container (10) with the channel(s) (17) thereof communicating with said peripheral channel (15), a separate closure member (18) being provided for each area defined by one or more channel section members (16) and the communicating peripheral channel (15) with the skirts (19) thereof depending downwardly thereinto.

3. An apparatus as claimed in claim 1 or 2 characterised in that the or each closure member (18) is formed with at least one opening (20) therein for locating apparatus (28, 29) to be in communication with the interior of the container (10).

4. An apparatus as claimed in any preceding claim characterised in that the container (10) comprises a tank fabricated of concrete, formed in situ.

5. An apparatus as claimed in any preceding claim characterised in that at least said channel (15,17) are lined with or formed of synthetic plastics material.

6. An apparatus as claimed in claim 1, 2 or 3 characterised in that the container (10) comprises a tank fabricated of synthetic plastics material.

7. A closure and support device characterised by comprising a part-spherical member (34) for locating within a complementary part-spherical aperture (20) in a support member (18) and an equipment support platform (35) located on a planar surface at a truncated upper portion of the part-spherical member (34).

8. An apparatus as claimed in claim 3 characterised in that a closure and support device as claimed in claim 7 is located in the or each opening (20) in the closure member (18) which comprises said support member (18).

9. A method of biologically degrading waste material characterised by locating such waste material within a container (10) of an apparatus as claimed in any one of claims 1 to 6 inclusive or 8 and maintaining the pressure of gases produced by the method at a relatively low pressure by feeding such gases from an outlet (26) of the container (10) to storage vessel (27) exterior thereto.

10. A method as claimed in claim 9 characterised in that said low pressure is in the order of one eighth inch (3.22mm) water gauge.

Fig. 1

Fig. 2

Fig. 3

Fig. 4